**Europäisches Patentamt**

(19) **European Patent Office**

**Office européen des brevets**

(11) Veröffentlichungsnummer: **0 061 777**

**A2**

(12) **EUROPÄISCHE PATENTANMELDUNG**

(21) Anmeldenummer: **82102687.9**

(22) Anmeldetag: **30.03.82**

(51) Int. Cl.³: **A 61 B 3/16**
**A 61 B 5/00**

(30) Priorität: **31.03.81 DE 3112910**

(43) Veröffentlichungstag der Anmeldung:
**06.10.82 Patentblatt 82/40**

(84) Benannte Vertragsstaaten:
**AT CH DE FR GB LI NL SE**

(71) Anmelder: **Fraunhofer-Gesellschaft zur Förderung der angewandten Forschung e.V.**
**Leonrodstrasse 54**
**D-8000 München 19(DE)**

(72) Erfinder: **Bramm, Günter, Dipl.-Ing.**
**Luisenstrasse 49**
**D-8000 München 2(DE)**

(74) Vertreter: **Czybulka, Uwe, Dipl.-Phys**
**Patentanwälte Haft, Berngruber, Czybulka**
**Hans-Sachs-Strasse 5**
**D-8000 München 5(DE)**

(54) **Einrichtung zum Messen des Augeninnendrucks.**

(57) Die Erfindung bezieht sich auf eine Einrichtung zur Langzeitmessung des Augeninnendruckes nach Art der Impressionstonometrie. Die Einrichtung weist einen Sensor (1) in Form einer auf das Auge aufsetzbaren elastischen Kontaktlinse (5) auf, die auf ihrer Unterseite eine kleine abgerundete zentrische Erhebung (7) trägt. Auf die Unterseite des Sensors ist über die gesamte Fläche verteilt eine elektrische Leiterbahn (9) in Form einer Spirale aufgebracht. Die Oberseite der Kontaktlinse ist mit einer etwa durchbrochenen oder spiralenförmig verlaufenden elektrischen Leiterbahn (11, 11') versehen. Die elektrischen Leiter auf der Ober- und Unterfläche sind miteinander verbunden und bilden einen passiven Sensorschwingkreis (H1). Ferner ist ein aktiver Schwingkreis (H2) mit einem Hochfrequenzgenerator (13) vorgesehen. Dieser Schwingkreis ist vorzugsweise in ein Brillengestell (12) eingebaut, ebenso wie ein Dip-Meter (15) und ein Telemetriesender (17). Der Sensor (1) wird entsprechend des Augeninnendruckes verformt, so daß sich dadurch die Resonanzfrequenz des Sensorschwingkreises (H1) ändert. Diese Änderung kann entweder durch die sogennante Dip-Frequenz, d.h. einem Einbruch im Schwingungsspektrum des aktiven Schwingungskreises oder als Änderung der Schwingungsamplitude des aktiven, mit einer einzigen Frequenz betriebenen aktiven Schwingkreises festgestellt werden und entsprechenden Augeninnendrücken zugeordnet werden.

Fig.1

Fig.4

Croydon Printing Company Ltd.

Die Erfindung bezieht sich auf eine Einrichtung zum Messen des Augeninnendruckes gemäß dem Oberbegriff des Patentanspruches 1.

In den Industrienationen sind neben der Diabetes die Glaukomerkrankungen (der sog. Grüne Star) die häufigste Ursache für spätere Erblindung. Dabei ist der erhöhte Augeninnendruck ein gemeinsames Symptom dieser Krankheitsgruppe. Die Messung des Augeninnendruckes ist daher zur Früherkennung und Kontrolle der Therapie, neben anderen Verfahren, eine der wichtigsten Aufgaben der Augenheilkunde. Diese Druckmessung ist dabei von großer Bedeutung sowohl bei glaukomverdächtigen, als auch bei schon glaumkomerkrankten Augen.

Für diese Druckmessung stehen heute Verfahren und Geräte zur Verfügung, die als ausgereift gelten können.

Die am häufigsten gebräuchlichen Einrichtungen arbeiten vorwiegend nach dem Prinzip der Abplattung der Hornhaut als Folge der Anwendung eines mechanischen Druckes (Applanationstonometrie).

Bei den Applanationstonometern, z. B. bei dem nach Goldmann, wird ein Stempel auf das Auge aufgesetzt und die Größe der Kraft bestimmt, die zur Abplattung einer bestimmten Fläche notwendig ist. Aufgrund einfacher geometrischer Zusammenhänge ist die Flächenpressung, die sich aus der Berührungsfläche und der Andruckkraft ergibt, ein Maß für den im Augeninneren herrschenden Druck, wenn die elastischen Eigenschaften der Hornhaut vernachlässigt werden können.

Bei Luftstrahltonometern neuerer Bauart wird ein kräftiger Luftstrom gegen das Auge gerichtet und die Verformung der Augenoberfläche optisch vermessen. Auch hier ist die Abplattung der Augenhornhaut ein Maß für den im Auge herrschenden Innendruck. Als Vorteil kann bei dieser Messung angesehen werden, daß sie ohne mechanische Berührung erfolgt. Allerdings wird aber der erforderliche starke Luftstrom vom Patienten als sehr unangenehm empfunden.

Außer den Applanationstonometern sind noch Impressionstonometer gebräuchlich, die nach folgendem Prinzip arbeiten: Hierbei wird die Tiefe der Eindellbarkeit der Hornhaut gemessen, die durch einen mit einem bekannten Gewicht belasteten Metallstempel hervorgerufen wird. Der Zusammenhang zwischen Meßwert und Augeninnendruck ergibt sich aus den sogenannten tonometrischen Tafeln, deren Tabellenwerte am enukleierten Auge bestimmt wurden.

Obwohl die eben beschriebenen diskontinuierlichen Methoden apparativ außerordentlich verfeinert worden sind, gibt es bisher noch kein Gerät, das in der Lage wäre, über einige Stunden, vor allem aber während des Schlafes des Patienten, den Augeninnendurck kontinuierlich zu messen. Solche Langzeitmessungen können jedoch von erheblicher klinischer Bedeutung sein. Hiermit könnte eine bessere Kenntnis über die tagesrhythmischen Schwankungen des Augeninnendruckes bei gesunden und erkrankten Augen gewonnen werden. Darüber hinaus wäre es möglich, mit einem solchen Gerät erstmals auch den Druckverlauf glaukomativer Augen während des Schlafes zu untersuchen und genauere Erkenntnisse über das Auftreten gefährlicher Druckspitzen zu erhalten. Gerade solche kontinuierlichen Messungen hinsichtlich der Druckschwankungen und Druckspitzen wären insbesondere bei Problemfällen auch zur Abstimmung der medikamentösen Behandlung wünschenswert. So ist z. B. der Wirkungsverlauf von Augendruck senkenden, pharmazeutischen Erzeugnissen nicht bekannt, bzw. es existieren hierüber nur Vermutungen.

Ähnliche Sachverhalte liegen vor bei den zum Teil unbekannten Mechanismen von Medikamentierungsdepots für Augendruck senkende Mittel.

Druckschwankungen während des Schlafes waren bisher überhaupt nicht diagnostizierbar. Deshalb kann man vermuten, daß sie auch bei Patienten aufgetreten sind, die tagsüber normale Druckwerte zeigten, trotzdem aber typische Glaukomschäden (z.B. Gesichtsfeldausfälle) aufweisen. Darüber hinaus ist eine Reihe von Fällen bei Augen-Hochdruck-Patienten bekannt, bei denen gerade nachts eine Erblindung auftritt. Über die Ursache gibt es bisher nur die Vermutung, daß Reaktionen auf Träume die Ursache des Druckanstiegs sein können.

Das bisher einzige bekannte Verfahren, das zumindest über einen geringen Zeitraum von Minuten kontinuierliche Ergebnisse liefert - die sogenannte Tonographie - arbeitet nach folgendem Prinzip: Ähnlich wie bei dem zuletzt beschriebenen Impressionstonometer, jedoch mit vollkommen anderer Aufgabenstellung, wird ein kleiner Stempel auf das Auge aufgedrückt. Ziel der Messung ist es, aus dem zeitlichen Verlauf der Eindelltiefe die Abflußleichtigkeit des Kammerwassers bzw. den Kammerwasserabflußwiderstand zu bestimmen.

Die Messung ist jedoch äußerst schwierig, kann nur im Liegen oder bei gestütztem, sehr ruhigem Sitzen geschehen und erfordert auch für den Patienten äußerste Konzentrationen und Mitarbeit, da er die Augenlider und die Augen nicht bewegen darf.

Der Erfindung liegt die Aufgabe zugrunde, eine Einrichtung zum Messen des Augeninnendruckes anzugeben, mit der kontinuierliche Langzeitmessungen des Augeninnendruckes ohne

erhebliche Behinderung des Patienten vorgenommen werden können.

Diese Aufgabe ist gemäß der Erfindung durch die im kennzeichnenden Teil des Patentanspruches 1 angegebenen Merkmale gelöst.

Eine Einrichtung gemäß der Erfindung weist demnach einen Sensor in Form einer elastischen Kontaktlinse auf, mit der ein passiver Sensorschwingkreis integriert ist. Die Kontaktlinse weist auf der der Augenhornhaut zugewandten Unterfläche eine stempelartige Erhebung auf. Wird die Kontaktlinse auf das Auge aufgesetzt, so wird die Unterseite der Kontaktlinse durch den Tränenfilm auf der Augenhornhaut angezogen. Durch die Adhäsionskräfte zwischen Hornhaut und der Kontaktlinse wird die Erhebung der Kontaktlinse auf die Hornhaut gedrückt und dellt diese entsprechend ein. Die hierdurch hervorgerufene Verformung der Kontaktlinse ändert auch die geometrische Zuordnung der Einzelteile der einzelnen Teile des in der Kontaktlinse integrierten passiven Sensorschwingkreises.

Diese Änderungen werden mit Hilfe eines induktiv gekoppelten äußeren aktiven Schwingkreises mit einem Hochfrequenzgenerator festgestellt, der eine Hochfrequenz-Wechselspannung abgibt, die in den passiven Sensorschwingkreis eingekoppelt wird. Mit einer Auswerteschaltung werden dann die durch die Verformung des Sensors bei Augeninnendruckschwankungen hervorgerufenen Änderungen der elektrischen Eigenschaften des passiven Sensorschwingkreises erfaßt und anhand von Referenz- bzw. Eichmessungen entsprechenden Werten des Augeninnendruckes zugeordnet.

Die eigentliche Auswertung des Resonanzverhaltens der beiden Schwingkreise kann prinzipiell auf zwei Weisen geschehen:

1. In der Amplitude des aktiven Schwingungskreises zeigt sich ein Einbruch bei der Frequenz, bei der die Resonanzfrequenz des passiven Sensorschwingkreises liegt. Diese Einbruchsfrequenz, die sogenannte Dip-Frequenz, ändert sich entsprechend der Resonanzfrequenz des passiven Sensorschwingkreises, welche wiederum ein Maß für die Verformung des Sensors und damit für den Augeninnendruck ist. Diese Dip-Frequenz ist unabhängig von dem Kopplungsfaktor der beiden Schwingkreise, d. h. unabhängig vom Abstand zwischen dem passiven Sensorschwingkreis und dem aktiven Schwingkreis.

2. Wenn der aktive Schwingkreis mit konstanter Frequenz betrieben wird, so kann die sich mit der Resonanzfrequenz des passiven Sensorschwingkreises ändernde Spannungsamplitude des aktiven Schwingkreises zur Auswertung benutzt werden. Hierbei ist jedoch darauf zu achten, daß die Resonanzfrequenzen der beiden Schwingkreise nicht gleich sind, jedoch sehr dicht beieinanderliegen. Da die Amplitude jedoch vom Kopplungsfaktor, d. h. vom Abstand der beiden Schwingkreise voneinander abhängt, dürfte der Anwendungsbereich dieser Auswertungsmöglichkeit eingeschränkt werden.

Die Auswertung der in den obigen beiden Punkten angegebenen Meßverfahren kann automatisch erfolgen. Möglich ist auch eine Aufzeichnung der Schwingungen des aktiven Schwingkreises, aufgrund der dann eine z. B. manuelle Auswertung anhand der erfaßten Dip-Frequenzen oder Spannungsamplituden erfolgt.

Um die Bewegungsfreiheit des Patienten möglichst nicht einzuschränken, wird gemäß einer bevorzugten Ausführungsform der Erfindung der aktive Schwingkreis in ein Brillengestell eingearbeitet, das von dem Patienten während der Messung getragen wird. Die Zuführung der Hochfrequenzenergie kann hierbei entweder über ein mit dem Brillengestell verbundenen Kabel geliefert werden, oder es wird in das Brillengestell direkt eine Batterie eingebaut und über eine entsprechende Schaltung die Hochfrequenz direkt hier erzeugt. Auf diese Weise kann sich der Patient völlig frei bewegen. Zur Auswertung ist in dem Raum, in dem sich der Patient aufhält, ein Auswertegerät aufgestellt, zu dem die von der Schwingkreisanordnung gemessenen Signale über eine an sich bekannte Infrarot- oder Ultraschalltelemetriestrecke übertragen werden. Bei einer solchen Anordnung bestehen für den Patienten keinerlei Beschränkungen hinsichtlich der Bewegung innerhalb des Aufenthaltsraumes.

Mit einer Einrichtung gemäß der Erfindung ist es möglich, den Augeninnendruck kontinuierlich über lange Zeit zu verfolgen und zu messen, so z. B. auch während des Schlafes des Patienten. Der Sensor arbeitet danach nach dem bewährten Prinzip der Impressionstonometrie, behindert jedoch dabei den Patienten in keiner Weise, so daß die Meßwerte unter realistischen Bewegungsbedingungen gewonnen werden. Der eigentliche Sensor ist wartungsfrei, da er keine eigene Spannungsversorgung wie z. B. eine Batterie, aufweist. Außerdem ist er sehr klein. Da zum Sensor keinerlei Kabel führen, sind Fehlmessungen z. B. aufgrund von Kabelkräften oder Kabelbewegungen nicht möglich. Die Anordnung des aktiven Schwingkreises in einer Art Brillengestell gewährleistet eine geringe Störungsanfälligkeit der telemetrischen Übertragung der Meßdaten vom Sensor auf die Schaltung in dem Brillengestell. Durch die Infrarot- oder Ultraschalltelemetriestrecke vom Brillengestell zum Auswertegerät kann sich der Patient innerhalb eines Raumes völlig frei bewegen. Der hierfür notwendige zusätzliche Schaltungsaufwand ist minimal und kann im Brillengestell untergebracht werden. Die Sensoren können weiterhin ohne großen Fertigungsaufwand hergestellt werden. Ein Austausch der Sensoren ist unproblematisch. Außerdem können sie z. B. mit Dampf oder Lösungen einwandfrei sterilisiert werden.

Weitere Ausgestaltungen und Vorteile der Erfindung gehen aus den Unteransprüchen in Verbindung mit der nachfolgenden Beschreibung hervor, in der zwei Ausführungsbeispiele anhand der Zeichnung näher erläutert sind. In der Zeichnung stellen dar:

Figur 1 einen Querschnitt durch einen als Kontaktlinse ausgebildeten Sensor für eine Einrichtung zum Messen des Augeninnendruckes gemäß der Erfindung mit einer schematischen Darstellung der auf den Sensor wirkenden Kräfte;

Figur 2 eine Aufsicht auf die Unterseite des in Figur 1 dargestellten Sensors;

Figur 3 eine Aufsicht auf die Oberfläche eines als Kontaktlinse ausgebildeten Sensors gemäß einem zweiten Ausführungsbeispiel;

Figur 4 eine schematische Darstellung aller Komponenten einer Einrichtung zur Augeninnendruckmessung gemäß der Erfindung;

Figur 5 ein mit einer Einrichtung gemäß Figur 4 aufgenommenes Meßsignal;

Figur 6 ein schematisches Diagramm zur Bestimmung des Augeninnendruckes aufgrund der von der Einrichtung gemäß der Erfindung aufgenommenen Signale.

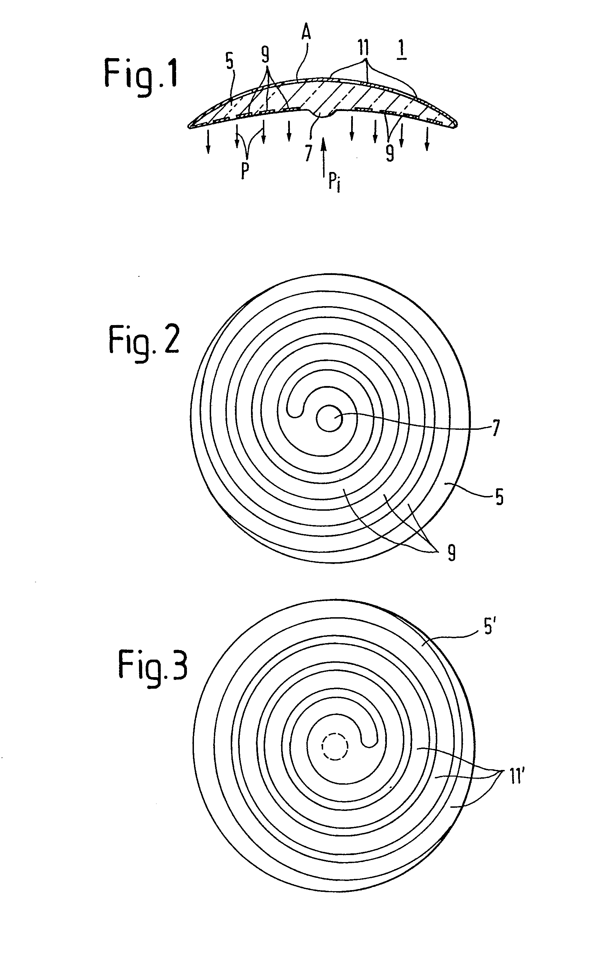

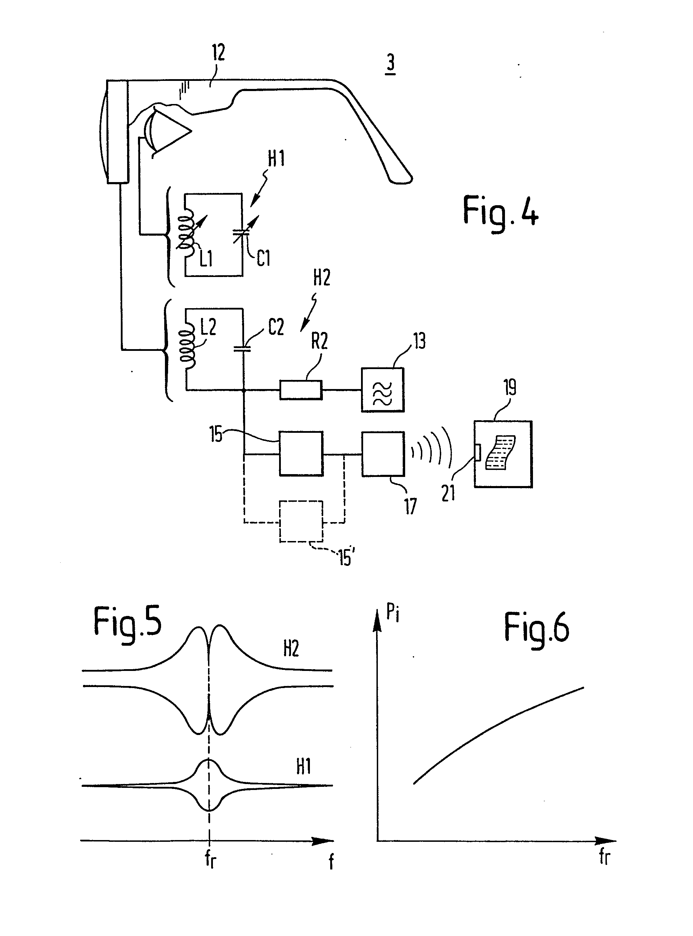

In Figur 1 ist im Querschnitt ein Sensor 1 für eine in ihrer Gesamtheit in Figur 4 gezeigte Einrichtung 3 zur Messung des Augeninnendruckes dargestellt. Der Sensor 1 weist eine elastische Kontaktlinse 5, z.B. aus Weich-Silikon-Gummi auf. Der Durchmesser der Kontaktlinse beträgt z.B. 12 mm. Die Kontaktlinse 5 wird auf das Auge eines Patienten aufgesetzt und dort durch den Tränenfilm in üblicher Weise gehalten. Auf der Unterfläche der Kontaktlinse weist diese eine zentrale kleine Erhebung 7 auf, deren Durchmesser etwa zwischen 0,8 und 1,2 mm liegt und deren Erhebungshöhe etwa 0,5 mm beträgt. Um trotz der Erhebung auch bei langfristigem Tragen der Kontaktlinse gute Verträglichkeit zu gewährleisten, sind die Rundungsradien an der Erhebung etwa 0,2 mm.

In diese Kontaktlinse 5 ist nun ein passiver Hochfrequenzschwingkreis H1 integriert, der eine Induktivität L1 und Kapazität C1 aufweist; vgl. auch Figur 4.

Im einzelnen kann dieser Schwingkreis wie in den Figuren 1 und 2 ausgebildet sein. Auf der die Erhebung 7 tragenden Unterseite der Kontaktlinse 5 ist ein dünner elektrischer Leiter 9 in Spiralenform aufgebracht. Diese Spirale erstreckt sich praktisch über die gesamte untere Fläche der Kontaktlinse. Die Oberfläche der Kontaktlinse trägt eine dünne elektrische Schicht 11, die ebenfalls nahezu die gesamte Oberseite der Kontaktlinse bedeckt. Die elektrische Schicht kann etwa nicht dargestellte Durchbrechungen aufweisen, um so eine ausreichende Durchsichtigkeit der Kontaktlinsen zu gewährleisten. Die elektrischen Leiter 9 und 11 auf der Unter- bzw. Oberseite sind elektrisch miteinander verbunden, wie dies in Figur 2 angedeutet ist, so daß sie insgesamt den in Figur 4 schematisch dargestellten Schwingkreis H1 bilden. Die elektrischen Leiter 9 und 11 können z. B. aus einer sehr dünnen leitfähigen Folie bestehen, aus leitenden Materialien aufgedampft oder aus einer aufgedampften Folie ausgeschnitten sein.

Auf eine Kontaktlinse 5 gemäß Figur 3 ist auf der Oberseite ein elektrischer Leiter 11' in Form einer Spirale aufgebracht. Die Unterseite der dargestellten Kontaktlinse 5' ist so wie bei dem Ausführungsbeispiel zu Figur 1 ausgebildet. Die Spiralen auf der Oberfläche und Unterseite der Kontaktlinse sind ebenfalls elektrisch miteinander verbunden, so daß sie den in Figur 4 dargestellten Schwingkreis H1 bilden. Zur Ausbildung des Schwingkreises sind selbstverständlich auch andere geometrische Formen der elektrischen Leiterbahnen möglich, solange nur der durch die elektrischen Leiterbahnen auf der Kontaktlinse gebildete Schwingkreis eine geeignete Resonanzfrequenz aufweist, wie dies weiter unten beschrieben wird.

Die Kontaktlinse 5 und die Beschichtungen 9 und 11 bzw. 9 und 11' bilden insgesamt den Sensor 1.

Wird dieser Sensor wie eine Kontaktlinse auf das Auge eines Patienten aufgesetzt, so wird dessen Unterseite durch die in Figur 1 durch Pfeile P schematisch angedeuteten Adhäsionskräfte aufgrund des Tränenfilmes auf der Hornhaut des Patienten festgehalten. Entgegen diesen Adhäsionskräften P wirkt auf die Erhebung 7 aufgrund des Augeninnendruckes entsprechend der Eindelltiefe der Hornhaut im Bereich der Erhebung eine Kraft $P_i$, wie dies ebenfalls schematisch in Figur 1 angedeutet ist. Insgesamt wirkt damit auf den Sensor ein Biegemoment, das den Sensor in Abhängigkeit der Eindelltiefe der Erhebung in der Hornhaut verformt. Aufgrund dieser Verformung ändert sich auch die geometrische Beziehung der elektrischen Leiter auf der Unter- und Oberseite der Kontaktlinse und auch die geometrische Beziehung des jeweiligen elektrischen Leiters auf einer Seite der Kontaktlinse. Im Falle, daß auf beiden Seiten der Kontaktlinse Spiralen als elektri-

sche Leiter vorgesehen sind, wie dies bei dem Ausführungsbeispiel gemäß Figur 3 der Fall ist, entsteht durch die geometrische Verformung der beiden spiralförmigen Leiter eine erhebliche Änderung der Impedanz des Schwingkreises H1. Die Resonanzfrequenz dieses Schwingkreises wird dabei durch die Kapazität der Windungen der elektrischen Leiter gegeneinander und durch die von einem eingestrahlten, weiter unten zu beschreibenden Magnetfeld hervorgerufene Induktivität bestimmt. Insgesamt kann die Änderung der Resonanzfrequenz des Sensorschwingkreises H1 durch einen vorwiegend kapazitiven Effekt, d. h. durch Annähern zweier Leiterbahnen bzw. eines leitfähigen Teiles, oder durch einen vorwiegend induktiven Effekt, d.h. Verschieben eines elektrisch leitenden Stückes oder Spulenteiles, erzeugt werden. Bei entsprechender Geometrie der elektrischen Leiter des Sensorschwingkreises ist eine Unterstützung beider Effekte in bestimmten Bereichen denkbar. Es muß jedoch stets die Relativbewegung der Erhebung 7 gegenüber den Randzonen des Kontaktlinsensensors zur Messung benutzt werden, so daß die elektrischen Leiter auf der Unter- und Oberseite der Kontaktlinse 5 über die gesamte Fläche der Kontaktlinse reichen sollten.

Zur Erfassung der durch die erwähnten Biegemomente veränderlichen Resonanzfrequenz setzt der Patient eine Spezialbrille 13 auf, in der ein aktiver Schwingkreis, bestehend aus einem Kondensator C2, einer Induktivität L2, einem Widerstand R2 und einem Hochfrequenzgenerator 13, eingebaut ist. Dieser aktive Hochfrequenzschwingkreis ist in Figur 4 schematisch dargestellt.

Der Hochfrequenzgenerator kann durch eine hier nicht dargestellte Batterie, die ebenfalls in dem Brillengestell eingebaut ist, mit Energie versorgt werden.

Die Auswertung des Resonanzverhaltens des Sensorschwingkreises H1 kann auf mehrere Arten erfolgen.

Ist der aktive Hochfrequenzschwingkreis H2 eingeschaltet,
so zeigt sich ein Einbruch in der Amplitude dieses aktiven
Schwingkreises H2 bei der jeweiligen Resonanzfrequenz des
Sensorschwingkreises H1. Dies ist schematisch in Figur 5
dargestellt, bei der im oberen Teil die Frequenzverteilung
des aktiven Schwingkreises und im unteren Teil die Frequenzverteilung des passiven Schwingkreises H1 dargestellt sind.
Bei der Resonanzfrequenz $f_r$ des Sensorschwingkreises H1 ist
deutlich der erwähnte Einbruch in der Amplitude des aktiven
Schwingkreises H2 zu erkennen. Die Resonanzfrequenz $f_r$, bei
der dieser Einbruch stattfindet, wird als sogenannte Dip-
Frequenz bezeichnet. Diese Dip-Frequenz ändert sich, wie
oben erwähnt, in Abhängigkeit des Biegemomentes auf den
Sensor 1 und damit auch in Abhängigkeit des jeweiligen
Augeninnendruckes $p_i$. Diese Dip-Frequenz ist unabhängig
von dem Kopplungsfaktor der beiden Induktivitäten L1 und L2,
d. h. unabhängig vom Abstand der beiden Schwingkreise H1
und H2.

Eine andere Möglichkeit der Erfassung der Resonanzfrequenzänderungen des Sensorschwingkreises H1 kann z. B. dadurch
erfolgen, daß der aktive Schwingkreis H2 mit konstanter
Frequenz betrieben wird. Je nach der Resonanzfrequenz des
passiven Sensorschwingkreises H1 kann dann die sich hierdurch ändernde Spannungsamplitude des aktiven Schwingkreises H2 zur Auswertung benutzt werden. Selbstverständlich
sollen die beiden Schwingkreise nicht auf der gleichen Resonanzfrequenz liegen, jedoch eng benachbarte Resonanzfrequenzen aufweisen, so daß eine wirkungsvolle Auswertung
möglich ist. Da die durch die jeweilige Resonanzfrequenz
des passiven Schwingkreises beeinflußte Amplitude des
aktiven Schwingkreises H2 jedoch außerdem vom Kopplungsfaktor, d.h. vom Abstand der beiden Schwingkreise H1 und H2 abhängt, und der Kopplungsfaktor nur schwierig konstant zu halten ist, ist der Anwendungsbereich dieser Auswertung eingeschränkt. Demgemäß wird zweckmäßig die oben erwähnte Auswertung mit Hilfe der Dip-Frequenz verwendet werden.

Für diese Auswertung ist mit dem aktiven Schwingkreis ein sogenanntes, an sich bekanntes Dip-Meter verbunden, mit dem die oben erwähnte Dip-bzw. Einbruchsfrequenz in der gewobbelten Frequenz des Hochfrequenzgenerators 13 bestimmt wird. Auch dieses Dip-Meter ist in die Spezialbrille 13 integriert, ebenso wie ein mit dem Ausgang des Dip-Meters verbundener Telemetriesender 17. Dieser Sender 17 ist z. B. ein Infrarot- oder Ultraschallsender, dessen Ausführung an sich bekannt ist und daher hier nicht näher beschrieben werden muß. Mit dem Sender 17 werden die von dem Dip-Meter 15 erfaßten Dip-Frequenzwerte zu einem stationären Auswertegerät 19 übertragen und dort von einem Empfänger 21 empfangen. In dem Auswertegerät 19 werden dann die empfangenen Dip-Frequenz-Werte jeweils entsprechenden Augeninnen- drücken $p_i$ zugeordnet. Eine solche Zuordnung kann z. B. aufgrund von Eichmessungen erfolgen, in denen eine entsprechende Beziehung zwischen dem Augeninnendruck $p_i$ und der Dip-Frequenz $f_r$ vermessen worden ist, wie diesen schematisch in Figur 6 dargestellt ist. Diese Zuordnung hängt wesentlich von der Geometrie des Sensors 1 ab. Die von dem Auswertegerät 19 ausgewerteten Ergebnisse können z. B. ausgedruckt, aufgezeichnet oder anderswie gespeichert werden.

Im übrigen ist es selbstverständlich möglich,anstelle der Dip-Frequenz-Werte die gewobbelte Frequenz des Hochfrequenzgenerators 13 zum Auswertegerät zu übertragen. Dieses Frequenzspektrum kann dort dann z. B. aufgezeichnet werden, wonach anhand dieser Aufzeichnung eine manuelle oder automatische Auswertung erfolgt. Anstelle einer solchen manuellen Auswertung können dem Auswertegerät selbst ein Dip-Meter enthalten sein, dessen Ausgangswerte in üblicher Form registriert werden.

Das stationäre Auswertegerät kann selbstverständlich durch entsprechende Programmierung eines Mikroprozessors verfeinert werden. So kann z. B. die Kennlinie zwischen Augeninnendruck und Dip-Frequenz linearisiert werden. Außerdem können für die Auswertung Störeffekte, die z. B. durch das Schließen der Augenlider oder durch den Vorgang des unwillkürlichen Blinzelns eintreten eliminiert werden.

Kriterium für die Erkennung solcher Störeffekte kann z. B. der rasche, kurzfristige Druckanstieg am Sensor sein. Dieser Störeffekt kann dann völlig ausgeblendet oder durch Rechnung korrigiert werden. Auf jeden Fall kann man mit einer solchen Einrichtung eine aufschlußreiche Langzeitmessung des Augeninnendruckes vornehmen.

Wird eine Auswertung entsprechend der obengenannten zweiten Art gewählt, so wird anstelle des Dip-Meters ein Spannungsdetektor 15' verwendet, der die Amplitudenschwankungen des in diesem Falle mit einer konstanten Frequenz betriebenen aktiven Schwingkreises H2 erfaßt. Diese Möglichkeit ist in Figur 4 schematisch gestrichelt dargestellt.

Fraunhofer-Gesellschaft zur Förderung der angewandten
Forschung e. V. , Leonrodstr. 54, 8000 München 19

Einrichtung zum Messen des Augeninnendruckes

P a t e n t a n s p r ü c h e

1. Einrichtung zum Messen des Augeninnendruckes nach
Art der Impressionstonometrie, gekennzeichnet durch
folgende Merkmale:

einen Sensor (1), bestehend aus einer auf die Augenhornhaut aufsetzbaren elastischen Kontaktlinse (5),
die auf ihrer, der Hornhaut zugewandten Unterseite
eine stempelartige, auf die Hornhaut drückende Erhebung (7) aufweist, sowie aus einem mit der Kontaktlinse integrierten, passiven Sensorschwingkreis (H1
aus 9, 11, 11');

einen aktiven Schwingkreis (H2) mit einem Hochfrequenzgenerator (13), der mit dem Sensorschwingkreis (H1) induktiv gekoppelt ist;

eine Auswerteschaltung (15, 17, 19, 21) zum Er
fassen der durch die Verformung des Sensors (1)
bei Augeninndendruckschwankungen hervorgerufenen
Änderungen der elektrischen Eigenschaften des
Sensorschwingkreises (H1).

2.  Einrichtung nach Anspruch 1, dadurch gekennzeichnet,
    daß auf der Kontaktlinse (5) des Sensors (1) eine
    elektrische Leiteranordnung (9, 11, 11'9 mit einer
    solchen Ausgestaltung der Leiterteile vorgesehen ist,
    daß ein passiver Schwingkreis (Sensorschwingkreis H1)
    entsteht.

3.  Einrichtung nach Anspruch 2, dadurch gekennzeichnet,
    daß in die Kontaktlinse (5) an der dem Auge zugewandten
    Seite eine elektrische Leiterbahn (9) in Spiralform eingeleg
    ist, die mit einer elektrischen Leiteranordnung (11) auf
    der Oberseite der Kontaktlinse (5) zur Bildung des Sensorschwingkreises (H1) verbunden ist.

4.  Einrichtung nach Anspruch 3, dadurch gekennzeichnet,
    daß auf der Oberseite der Kontaktlinse ein elektrischer
    Leiter (11') in Form einer Spirale aufgebracht ist.

5.  Einrichtung nach einem der vorhergehenden Ansprüche,
    dadurch gekennzeichnet, daß die elektrischen Leiter-
    anordnungen (9, 11, 11!) auf der Kontaktlinse zur
    Bildung des Sensorschwingkreises (H1) über die gesamte
    Fläche der Kontaktlinse (5) verteilt sind.

6.  Einrichtung nach einem der vorhergehenden Ansprüche,
    dadurch gekennzeichnet, daß der aktive Schwingkreis (H2)
    ein um die durch Durchbiegungen des Sensors variable
    Resonanzfrequenz ($f_r$) liegendes Frequenzspektrum ab-

gibt, und daß die Auswerteschaltung (15, 17, 19, 21) eine
Schaltung (Dip-Meter 15) zum Erfassen der dem Augeninnendruck zuzuordnenden Resonanzfrequenz (Dip-Frequenz
$f_r$) des Sensorschwingkreises (H1) aufweist.

7. Einrichtung nach einem der vorhergehenden Ansprüche 1
bis 5, dadurch gekennzeichnet, daß der aktive Schwingkreis (H2) eine konstante Frequenz abgibt, daß diese
Frequenz nahe der in Abhängigkeit des Augeninnendruckes
($p_i$) veränderlichen Resonanzfrequenz ($f_r$) des Sensorschwingkreises ($H_1$) liegt, und daß die Auswerteschaltung eine Schaltung (15') zum Erfassen der spezifischen
Augeninnendrücken zuzuordnenden Amplitudenänderungen
der Schwingung des aktiven Schwingkreises ($H_2$) aufweist.

8. Einrichtung nach einem der Ansprüche 6 und 7, dadurch
gekennzeichnet, daß die Auswerteschaltung (15, 17, 19,
21) einen Telemetriesender (17) zum drahtlosen Übertragen der erfaßten Meßwerte an ein, einen Empfänger (21)
aufweisendes Auswertegerät (19) aufweist.

9. Einrichtung nach einem der vorhergehenden Ansprüche,
dadurch gekennzeichnet, daß die Einrichtung eine von
einem Patienten aufsetzbare Spezialbrille (12) aufweist, in der der aktive Schwingkreis (H2) und zumindest Teile (15, 17) der Auswerteschaltung integriert
sind.

10. Einrichtung nach Anspruch 9, dadurch gekennzeichnet,
daß in der Brille der aktive Schwingkreis (H2) mit dem
eine eigene Stromversorgung aufweisenden Hochfrequenzgenerator (13), eine Schaltung (15, 15') zum Erfassen
der sich in Abhängigkeit des Augeninnendruckes ändernden elektrischen Eigenschaften des Sensorschwingkreise
(H1) und ein Telemetriesender (17) zum Übertragen der erfaßten Meßwerte an ein stationäres Auswertegerät (19)
eingebaut sind.

Fig.1

Fig. 2

Fig.3

Fig. 4

Fig.5

Fig.6